Europäisches Patentamt

European Patent Office

Office européen des brevets

(11) Publication number: 0 546 045 B1

# EUROPEAN PATENT SPECIFICATION

(45) Date of publication of patent specification: 04.01.95

(51) Int. Cl.⁶: **C12N 9/42**, C12P 21/00, D21C 9/10, C12N 15/56, //C12S3/08

(21) Application number: 91915880.8

(22) Date of filing: 23.08.91

(86) International application number: PCT/DK91/00242

(87) International publication number: WO 92/03540 (05.03.92 92/06)

(54) ENZYMES WITH XYLANOLYTIC ACTIVITY.

(30) Priority: 24.08.90 PCT/DK90/00220
22.02.91 DK 309/91

(43) Date of publication of application:
16.06.93 Bulletin 93/24

(45) Publication of the grant of the patent:
04.01.95 Bulletin 95/01

(84) Designated Contracting States:
AT BE CH DE DK ES FR GB GR IT LI LU NL SE

(56) References cited:
WO-A-89/08738

Viikarie L. et al. "Fourth International Symposium on Wood and Pulping Chemistry, Paris (1987), p. 151-154

Chauvet J.-M. et al. Fourth International Symposium on Wood and Pulping Chemistry, Paris (1987), p. 325-327

Biotec0nology and Bioengineering, vol. 32, July 1988 M.G. Paice et al. s. pp. 235-239

(73) Proprietor: NOVO NORDISK A/S
Novo Allé
DK-2880 Bagsvaerd (DK)

(72) Inventor: ANKER, Lisbeth
Athensvej 16
DK-2300 Copenhagen S (DK)
Inventor: BISGAARD-FRANTZEN, Henrik
Sandkrogen 27
DK-2800 Lyngby (DK)
Inventor: HALKIER, Torben
Vodroffsvej 6G
DK-1900 Frederiksberg C (DK)

Rank Xerox (UK) Business Services
(3.10/3.09/3.3.3)

```
                         SEQUENCE LISTING

    (1) GENERAL INFORMATION:

        (i) APPLICANT: NOVO NORDISK A/S, NN

        (ii) TITLE OF INVENTION: NOVEL ENZYMES

        (iii) NUMBER OF SEQUENCES: 8

    (2) INFORMATION FOR SEQ ID NO:1:

        (i) SEQUENCE CHARACTERISTICS:
            (A) LENGTH: 201 amino acids
            (B) TYPE: amino acid
            (C) STRANDEDNESS: single
            (D) TOPOLOGY: linear

        (ii) MOLECULE TYPE: protein

        (vi) ORIGINAL SOURCE:
            (A) ORGANISM: Bacillus pumilus
            (B) STRAIN: IPO

        (x) PUBLICATION INFORMATION:
            (A) AUTHORS: Fukusaki, E.
                         Panbangred, W.
                         Shinmyo, A.
                         Okada, H.
            (B) TITLE: The complete nucleotide sequence of the
                xylanase gene (xynA) of Bacillus pumilus
            (C) JOURNAL: FEBS Lett.
            (D) VOLUME: 171
            (E) ISSUE: 2
```

(F) PAGES: 197-201
(G) DATE: 1984

(xi) SEQUENCE DESCRIPTION: SEQ ID NO:1:

```
Arg Thr Ile Thr Asn Asn Glu Met Gly Asn His Ser Gly Tyr Asp Tyr
1               5                   10                  15

Glu Leu Trp Lys Asp Tyr Gly Asn Thr Ser Met Thr Leu Asn Asn Gly
            20                  25                  30

Gly Ala Phe Ser Ala Gly Trp Asn Asn Ile Gly Asn Ala Leu Phe Arg
            35                  40                  45

Lys Gly Lys Lys Phe Asp Ser Thr Arg Thr His His Gln Leu Gly Asn
        50                  55                  60

Ile Ser Ile Asn Tyr Asn Ala Ser Phe Asn Pro Ser Gly Asn Ser Tyr
65                  70                  75                  80

Leu Cys Val Tyr Gly Trp Thr Gln Ser Pro Leu Ala Glu Tyr Tyr Ile
                85                  90                  95

Val Asp Ser Trp Gly Thr Tyr Arg Pro Thr Gly Ala Tyr Lys Gly Ser
                100                 105                 110

Phe Tyr Ala Asp Gly Gly Thr Tyr Asp Ile Tyr Glu Thr Thr Arg Val
            115                 120                 125

Asn Gln Pro Ser Ile Ile Gly Ile Ala Thr Phe Lys Gln Tyr Trp Ser
        130                 135                 140

Val Arg Gln Thr Lys Arg Thr Ser Gly Thr Val Ser Val Ser Ala His
145                 150                 155                 160
```

```
Phe Arg Lys  rp Glu Ser Leu Gly Met Pro Met Gly Lys Met Tyr Glu
            165             170             175


Thr Ala Phe Thr Val Glu Gly Tyr Gln Ser Ser Gly Ser Ala Asn Val
            180             185             190


Met Thr Asn Gln Leu Phe Ile Gly Asn
            195             200
```

(2) INFORMATION FOR SEQ ID NO:2:

    (i) SEQUENCE CHARACTERISTICS:
        (A) LENGTH: 28 amino acids
        (B) TYPE: amino acid
        (D) TOPOLOGY: linear

    (v) FRAGMENT TYPE: N-terminal

    (vi) ORIGINAL SOURCE:
        (A) ORGANISM: Bacillus pumilus
        (B) STRAIN: DSM 6124

    (xi) SEQUENCE DESCRIPTION: SEQ ID NO:2:

```
Glu Thr Ile Tyr Asp Asn Arg Ile Gly Arg His Thr Gly Tyr Asp Phe
1               5               10              15


Glu Leu Trp Lys Asp Tyr Gly Asn Thr Ser Met Ile
            20              25
```

(2) INFORMATION FOR SEQ ID NO:3:

    (i) SEQUENCE CHARACTERISTICS:
        (A) LENGTH: 31 amino acids

```
        (B) TYPE: amino acid
        (D) TOPOLOGY: linear


    (v) FRAGMENT TYPE: internal


    (vi) ORIGINAL SOURCE:
        (A) ORGANISM: Bacillus pumilus
        (B) STRAIN: DSM 6124


    (xi) SEQUENCE DESCRIPTION: SEQ ID NO:3:


    Ser Ala Ser Trp Asn Asn Ile Gly Asn Ala Leu Phe Arg Lys Gly Lys
    1                   5                   10                  15


    Lys Phe Asp Ser Thr Lys Thr His His Gln Leu Gly Asn Ile Asp
                    20                  25                  30


(2) INFORMATION FOR SEQ ID NO:4:


    (i) SEQUENCE CHARACTERISTICS:
        (A) LENGTH: 104 amino acids
        (B) TYPE: amino acid
        (D) TOPOLOGY: linear


    (v) FRAGMENT TYPE: C-terminal


    (vi) ORIGINAL SOURCE:
        (A) ORGANISM: Bacillus  pumilus
        (B) STRAIN: DSM 6124


    (xi) SEQUENCE DESCRIPTION: SEQ ID NO:4:


    Ile Val Glu Ser Trp Gly Thr Tyr Arg Pro Thr Gly Thr Tyr Lys Trp
    1                   5                   10                  15
```

Ser Phe Tyr Ala Asp Gly Gly Thr Tyr Asp Ile Tyr Glu Thr Leu Arg
        20               25           30

Val Asn Gln Pro Ser Ile Ile Gly Asp Ala Thr Phe Lys Gln Tyr Val
        35               40           45

Ser Val Arg Gln Thr Lys Arg Thr Ser Gly Thr Val Ser Val Ser Glu
        50               55           60

His Phe Lys Lys Trp Glu Gly Leu Tyr Met Pro Met Gly Lys Met Tyr
65               70           75              80

Glu Thr Ala Leu Thr Val Glu Gly Tyr Arg Ser Thr Gly Ser Ala Asn
          85            90           95

Val Met Thr Asn Gln Leu Met Ile
          100

(2) INFORMATION FOR SEQ ID NO:5:

    (i) SEQUENCE CHARACTERISTICS:
        (A) LENGTH: 16 amino acids
        (B) TYPE: amino acid
        (C) STRANDEDNESS: single
        (D) TOPOLOGY: linear

    (ii) MOLECULE TYPE: peptide

    (v) FRAGMENT TYPE: internal

    (xi) SEQUENCE DESCRIPTION: SEQ ID NO:5:

Ile Val Glu Ser Trp Gly Thr Tyr Arg Pro Thr Gly Thr Tyr Lys Gly
1            5               10            15

(2) INFORMATION FOR SEQ ID NO:6:

    (i) SEQUENCE CHARACTERISTICS:
        (A) LENGTH: 23 amino acids
        (B) TYPE: amino acid
        (C) STRANDEDNESS: single
        (D) TOPOLOGY: linear

    (ii) MOLECULE TYPE: peptide

    (v) FRAGMENT TYPE: internal

    (xi) SEQUENCE DESCRIPTION: SEQ ID NO:6:

Val Asn Gln Pro Ser Ile Ile Gly Asp Ala Thr Phe Lys Gln Tyr Val
1               5                   10                  15

Ser Val Arg Gln Thr Lys Arg
          20

(2) INFORMATION FOR SEQ ID NO:7:

    (i) SEQUENCE CHARACTERISTICS:
        (A) LENGTH: 8 amino acids
        (B) TYPE: amino acid
        (C) STRANDEDNESS: single
        (D) TOPOLOGY: linear

    (ii) MOLECULE TYPE: peptide

    (v) FRAGMENT TYPE: internal

    (xi) SEQUENCE DESCRIPTION: SEQ ID NO:7:

```
His Phe Lys Lys Xaa Glu Gly Leu
1               5
```

(2) INFORMATION FOR SEQ ID NO:8:

    (i) SEQUENCE CHARACTERISTICS:

        (A) LENGTH: 5 amino acids

        (B) TYPE: amino acid

        (C) STRANDEDNESS: single

        (D) TOPOLOGY: linear

    (ii) MOLECULE TYPE: peptide

    (v) FRAGMENT TYPE: internal

    (xi) SEQUENCE DESCRIPTION: SEQ ID NO:8:

```
Thr Val Ala Gly Tyr
1               5
```

**Description**

**FIELD OF THE INVENTION**

This invention relates to novel enzymes possessing xylanolytic activity. More specifically, the invention relates to novel xylanases obtainable from strains of Bacillus pumilus, a process for their preparation, and the use of these xylanases for treatment of lignocellulosic pulp.

**BACKGROUND ART**

Members of Bacillus pumilus are known and described in the literature, and it is known that they are able to produce xylanases.

A specific isolate from soil in Thailand, B. pumilus IPO, has been thoroughly described by Panbangred et al. (1983); J. Agric. Biol. Chem.; 47(5), 957-963; and Fukusaki et al. (1984); FEBS Lett.; 171(2), 197-201. In JP Patent Specification No. 86039036, this B. pumilus IPO xylanase is provided by way of recombinant DNA technology. The specification mentions the use of this enzyme in the decomposition of xylan contained in animal feed or in clarification of muddy wines or fruit juices. There is given no indication of its usefulness in the treatment of lignocellulosic pulp.

**SUMMARY OF THE INVENTION**

Now a novel xylanase obtainable from a strain of B. pumilus has been elucidated. The xylanase of this invention possesses excellent properties for treatment of lignocellulosic pulp.

Accordingly, in its first aspect, the present invention provides a xylanase comprising specified partial amino acid sequences, and having immunochemical properties identical or partially identical to those of a xylanase derived from the strain DSM, No. 6124.

In another aspect, the invention provides a xylanase comprising specified partial amino acid sequences, and being obtainable from a strains of Bacillus pumilus.

The invention also provides a method of producing the xylanase, comprising cultivation of a strain of B. pumilus, or a mutant or a variant thereof, in a nutrient medium containing assimilable carbon and nitrogen together with other essential nutrient, followed by recovery of the desired enzyme.

In a further aspect, the invention provides a method of producing the xylanase, comprising isolating a DNA fragment encoding the xylanase; combining the DNA fragment with an appropriate expression signal in an appropriate plasmid vector; introducing the plasmid vector into an appropriate host either as an autonomously replicating plasmid or integrated into the chromosome; cultivating the host organism under conditions leading to expression of the xylanase; and recovering the xylanase from the culture medium.

Moreover, the invention relates to the use of the xylanase in the treatment of lignocellulosic pulp. In a more specific aspect, the invention provides a process for treatment of lignocellulosic chemical pulp, wherein the lignocellulosic pulp is treated with the xylanase at a pH of above 6.5, preferably above 7.5, whereafter the thus treated cellulosic pulp is treated with chlorine at an active chlorine multiple of 0.20 or less in the first chlorination stage.

**BRIEF DESCRIPTION OF DRAWINGS**

The present invention is further illustrated by reference to the accompanying drawings, in which:

Fig. 1 shows a comparison of the amino acid sequence of the B. pumilus IPO xylanase according to Fukusaki et al. (1984), op. cit. (the upper sequence; similar to Sequence No. 1, presented in the sequence list below) and the partial amino acid sequence of the xylanase of the invention (the lower sequence; similar to Sequence Nos. 2-3, presented in the sequence list below); the amino acids are indicated in the established one-letter code;

Fig. 2 shows the pH profile of a xylanase of the invention;

Fig. 3 shows the temperature profile of a xylanase of the invention; and

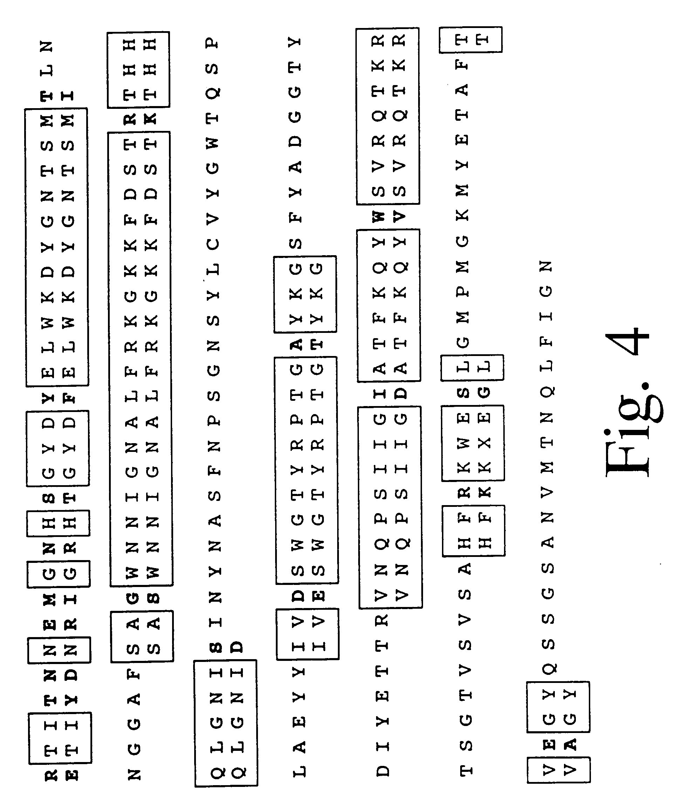

Fig. 4 shows a comparison of the amino acid sequence of the B. pumilus IPO xylanase according to Fukusaki et al. (1984), on. cit. (the upper sequence; similar to Sequence No. 1, presented in the sequence list below) and the partial amino acid sequence of the xylanase of the invention (the lower sequence; similar to Sequence Nos. 5-8, presented in the sequence list below); the amino acids are indicated in the established one-letter code.

## DETAILED DISCLOSURE OF THE INVENTION

The present invention provides a novel xylanase. This novel xylanase is especially useful for treatment of lignocellulosic pulp.

### The enzymes

The novel xylanase of the invention is obtainable from Bacillus pumilus, DSM No. 6124 (deposited on July 23, 1990 at Deutsche Sammlung von Mikroorganismen under the conditions of the Budapest Treaty), or mutants or variants thereof.

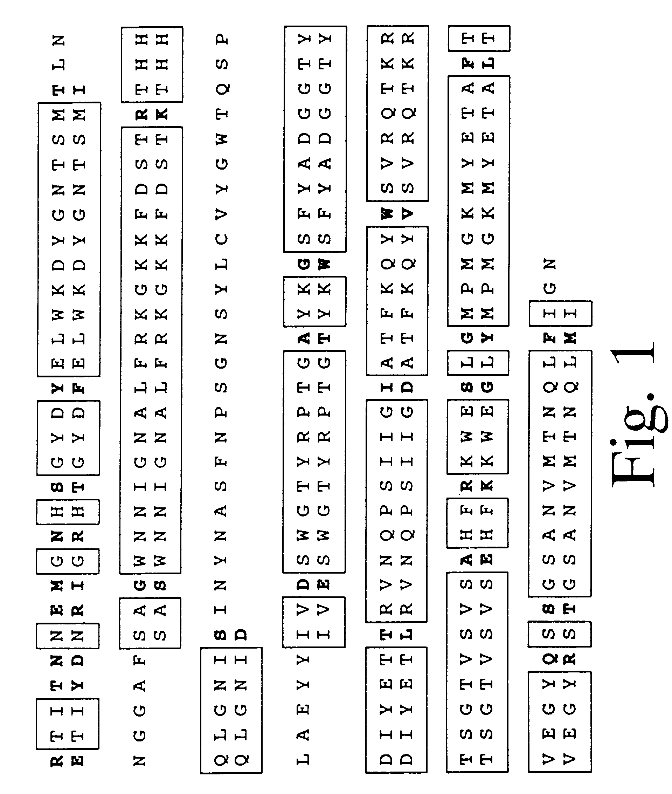

The xylanase of the invention comprises the amino acid sequences that appear from Fig. 1 and Sequence Nos. 2-4 of the sequence list. On the figure the upper amino acid sequence represents the B. pumilus IPO xylanase described by Fukusaki et al. (1984), op. cit. (Seq. No. 1), and the lower amino acid sequences represent sequences that are found to be held within the amino acid sequence of the xylanase of this invention (Seq. Nos. 2-4).

The lower sequence was found after proteolytic degradation of the xylanase. Among 163 amino acid residues determined, 26 are found to be different from the B. pumilus IPO xylanase amino acid sequence published. On the figure the positions that differ between the two sequences are shown in bold typing whereas positions with identical amino acid residues are outlined.

The xylanase of the invention has immunochemical properties identical or partially identical to those of a xylanase derived from the strain DSM, No. 6124.

The immunochemical properties can be determined immunologically by cross-reaction identity tests. The identity tests can be performed by the well-known Ouchterlony double immunodiffusion procedure or by tandem crossed immunoelectrophoresis according to N. H. Axelsen; Handbook of Immunoprecipitation-in-Gel Techniques; Blackwell Scientific Publications (1983), chapters 5 and 14. The terms "antigenic identity" and "partial antigenic identity" are described in the same book, chapters 5, 19 and 20.

The molecular weight of a xylanase of this invention has been estimated to approximately 22 kDa, as determined by SDS-PAGE.

The xylanase possesses xylanolytic activity in a pH range of from below pH 5 to above pH 11. At pH 5 more than 90% relative activity is determined. The pH optimum for the xylanase is in the range of from pH 5 to pH 7, around pH 6.

The temperature profile for the xylanase is very broad, and the xylanase has no distinct temperature optimum. The xylanase is found to be nearly as active at 60°C, with around 95% persisting relative activity, as at 40°C. The temperature optimum is found to be lower than 70°C, where only around 5% of the xylanase activity persist.

The pI for the purified xylanase was found to be within the range of from 8.0 to 9.5, around 8.8.

The amino acid composition of the xylanase has been determined after acid hydrolysis and it is found to differ from the amino acid composition of the B. pumilus IPO xylanase, especially in the content of serine and glycine, as shown in Table 1.

EP 0 546 045 B1

Table 1

| Amino acid | B. pumilus xylanase amino acid composition (mol-%) | B. pumilus xylanase amino acid composition according to molecular weight (residues) | B. pumilus IPO xylanase amino acid composition calculated from the amino acid sequence (residues) |
|---|---|---|---|
| | | | |
| Asx | 11.5 | 22 | 24 |
| Glx | 7.5 | 14 | 14 |
| Ser | 7.9 | 15 | 20 |
| Gly | 10.0 | 19 | 23 |
| His | 2.0 | 4 | 4 |
| Arg | 4.3 | 8 | 8 |
| Thr | 9.4 | 18 | 19 |
| Ala | 5.5 | 11 | 11 |
| Pro | 2.6 | 5 | 5 |
| Tyr | 7.6 | 15 | 16 |
| Val | 4.1 | 8 | 8 |
| Met | 2.7 | 5 | 6 |
| Cys | 0.5 | 1 | 1 |
| Ile | 4.6 | 9 | 10 |
| Leu | 5.4 | 10 | 8 |
| Phe | 4.4 | 8 | 9 |
| Lys | 5.9 | 11 | 9 |
| Trp | 4.1 | 8 | 6 |
| | | | |
| Total | 100.0 | 191 | 201 |

The preparation

The xylanase of the invention is producible by cultivation of a xylanase producing strain of Bacillus pumilus, preferably B. pumilus, DSM No. 6124, or a mutant or a variant thereof.

Xylanase producing B. pumilus strains can be cultivated under aerobic conditions in a nutrient medium containing assimilable carbon and nitrogen together with other essential nutrient. The medium can be composed in accordance with principles known in the art.

During cultivation, the cells secrete xylanase extracellularly, so the recovery of xylanase preferably includes separation of cell mass from the cell broth while avoiding cell lysis, e.g. by filtration or centrifugation.

The resulting cell-culture can be used as such, optionally after concentration e.g. by evaporation or ultrafiltration. If desired, the xylanase can be separated and purified to the desired degree by conventional methods, e.g. by column chromatography.

The xylanase is also obtainable by recombinant DNA technology by methods known in the art per se, e.g. isolating a DNA fragment encoding the xylanase; combining the DNA fragment with an appropriate expression signal in an appropriate plasmid vector; introducing the plasmid vector into an appropriate host (i.e. an Escherichia coli, or a member of the genus Bacillus, Aspergillus, or Streptomyces), either as an autonomously replicating plasmid or integrated into the chromosome; cultivating the host organism under

4

conditions leading to expression of the xylanase; and recovering the xylanase from the culture medium.

The xylanase also may be produced essentially as described in JP Patent Specification No. 86039036.

Treatment of lignocellulosic pulp

The xylanase of this invention is valuable for treatment of lignocellulosic pulp.

Xylan, a major component of plant hemicellulose, is a polymer of D-xylose linked by B-1,4-xylosidic bonds. Xylan can be degraded to xylose and xylo-oligomers by acid or enzymatic hydrolysis. Enzymatic hydrolysis of xylan produces free sugars without the by-products formed with acid (e.g. furans).

The pulp and paper industry is using xylanase compositions in the bleaching process to enhance the brightness of bleached pulps, to decrease the amount of chlorine used in the bleaching stages, and to increase the freeness of pulps in the recycled paper process [Eriksson. K.E.L. (1990); Wood Science and Technology, 24, 79-101.; Paice, M.G.; Bernier, R.; & Jurasek, L. (1988); Biotechnol. and Bioeng., 32, 235-239; Pommier, J.C.; Fuentes, J.L.; & Goma, G.(1989); Tappi Journal, 187-191].

Processes for treatment of lignocellulosic pulp are widely described in the literature, vide e.g. Paice. M.G. & Jurasek, L. (1984); Journal of Wood Chemistry and Technology, 4(2), 187-198; Noe, P. et al. (1986); Journal of Wood Chemistry and Technology, 6(2), 167-184; or EP Patent Application No. 386,888.

In relation to the use of the xylanase of this invention for treatment of lignocellulosic pulp, a particular process is described in International Patent Publication No. WO 91/02839.

The invention is further illustrated in the following examples which are not intended to be in any way limiting to the scope of the invention as claimed.

**EXAMPLE 1**

Production of Bacillus pumilus xylanase

A Bacillus pumilus culture DSM 6124 was maintained on A3-medium, at 37°C.

| A3-medium: | |
|---|---|
| | g/l |
| Peptone, Difco™ | 6.0 |
| Yeast Extract | 3.0 |
| Pepticase™ | 4.0 |
| Beef Extract | 1.5 |
| Glucose | 1.0 |
| Agar, Merck™ | 20.0 |
| H$_2$O | 1000 ml |
| pH adjustment to 7.3 before autoclaving. Autoclaving 25 min./121°C. | |

120 shake flasks with 150 ml XYL-8 medium each, inoculated from A3-agar slants, were cultivated for 4 days, 37°C, 250 rpm with approximately 2 cm amplitude.

| XYL-8 medium: | |
|---|---|
| | g/l |
| Bacto-peptone, Difco™ | 10.0 |
| Yeast extract | 10.0 |
| $K_2HPO_4$ | 15.0 |
| $MgSO_4.7H_2O$ | 0.5 |
| KCl | 0.1 |
| $FeSO_4.7H_2O$ | 0.01 |
| Beech xylan, Lenzing™ | 6.0 |
| pH adjustment to 7.0 before autoclaving. Autoclaving 25 min./121 °C. | |

The broth was centrifuged for half an hour at 4,000 rpm (SORVALL RC-3B centrifuge with a 6000 A rotor). The supernatant, 7.3 l, was filtered through a 10 $\mu$m nylon filter, and concentrated by ultrafiltration by means of a Pellicon equipment from Millipore, with a 10,000 MW cut-off membrane and washed 2 times with one volume of water. This resulted in 540 ml of concentrate. The concentrate was then lyophilized, whereby 8.8 g of powder were generated.

## EXAMPLE 2

Purification example

The xylanase was purified in seven steps.

Approximately 500 ml of fermentation broth were centrifuged at 5000 rpm for 30 min. The supernatant was then precipitated with 50% w/w ammonium sulfate. The precipitate was dissolved in 20 mM Tris-HCl buffer, pH 7.0, and desalted and concentrated until the conductivity was less than 1 mS/cm using a Filtron ultrafiltration module with 3 kD cut off membranes.

The concentrate was applied to a 25 ml anionexchanger (Q-sepharose® F.F.) equilibrated with 20 mM Tris-HCl buffer, pH 7.0.

The flow-trough was applied to a 25 ml cationexchanger (S-sepharose® F.F.) equilibrated with the same buffer. The column was eluted with a linear gradient consisting of 200 ml of the above buffer and 200 ml added 500 mM NaCl.

Most of the remaining proteases of the fractions with xylanase activity were removed by affinity chromatography at pH 7.0.

The flow-through was added ammonium sulfate to a concentration of 20% w/w and applied on a 10 ml Phenyl-Sepharose® CL-4B. The column was eluted with a linear gradient consisting of 750 ml 20 mM Tris-HCl buffer, pH 7.0, 20% w/w ammonium sulfate, and 750 ml of the same buffer without ammonium sulfate.

The fractions with xylanase activity were concentrated using an Amicon ultrafiltration unit 8200 with DDS GR90PP membranes. When the concentration of xylanase was approximately 100 mg/ml and the conductivity was 20 mS/cm, the xylanase started to crystallize.

The yield was near 100%. The crystals were dissolved in 20 mM Tris-HCl buffer, pH 7.0, 25% w/w glycerol.

## EXAMPLE 3

Method for determining xylanase activity

Xylanase activity was determined by assaying for reducing sugars released from Birch xylan (the XU method).

The assay is performed using 0.5% Birch xylan (ROTH GmbH Atr. 7500) prepared in 40 mM Britton-Robinson (J. Chem. Soc., 1931, p. 1451) buffer (heat treated 30 min. at 100 °C before use) as substrate. The assay is run for 20 min. at 40 °C using 0.100 ml Enzyme Solution I (35.1 g $Na_2HPO_4$, $2H_2O$; 40.0 g $KNaC_4H_4O_6$, $4H_2O$, suspended in 500 ml deionized $H_2O$; 42 ml concentrated $H_2SO_4$; 6.0 g $Na_2HAsO_4$, $7H_2O$; ad deionized water to a total volume of 1 litre) is added. Add 2.0 ml of deionized water and measure the absorbance on a spectrophotometer (PYE UniCAM PU8600UV/VIS, Phillips) at 250 nm. The reducing sugars are calculated from a standard curve prepared with xylose (40-400 $\mu$g/ml). One XU is equivalent to 1 nmol xylose released per second per millilitre or per gram of culture broth.

### EXAMPLE 4

pH characterization

The pH characterization of the xylanase was determined using the purified xylanase preparation according to Examples 1 and 2, and the XU method described in Example 3.

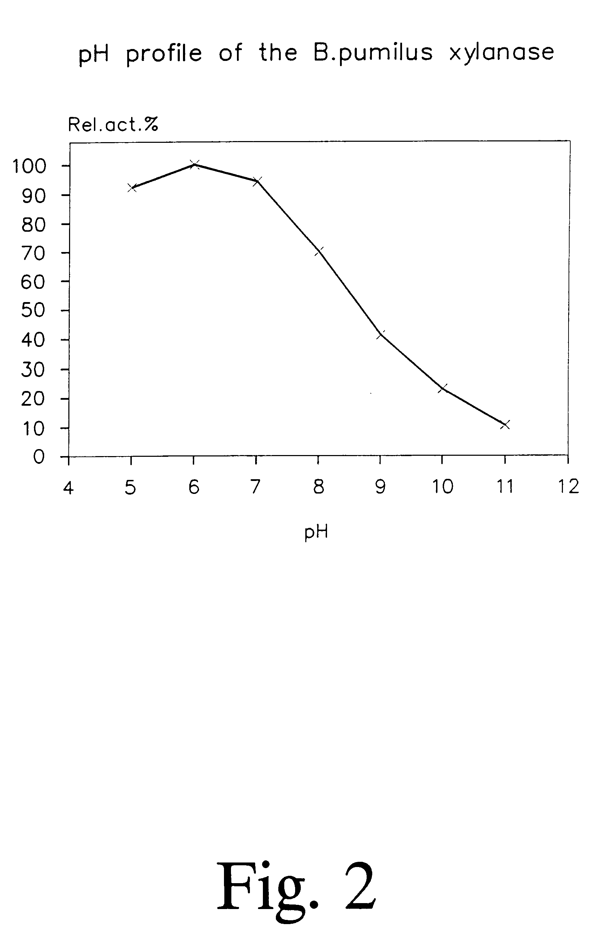

The characterization was performed in the range of from pH 5 to pH 11. All assays were performed for 20 min. at 40°C. The profile is presented in Fig. 2.

It appears from this characterization that the xylanase of the invention possesses xylanolytic activity from below pH 5 to above pH 11. At pH 5 a relative activity of more than 90% has been determined. At pH 9 a relative activity of more than 40% has been determined. At pH 11 a relative activity of more than 10% has been determined.

The pH optimum for the xylanase is in the range of from pH 5 to pH 7, around pH 6.

### EXAMPLE 5

Temperature characterization

The temperature characterization of the xylanase was determined using the purified xylanase preparation according to Examples 1 and 2, and the XU method described in Example 3.

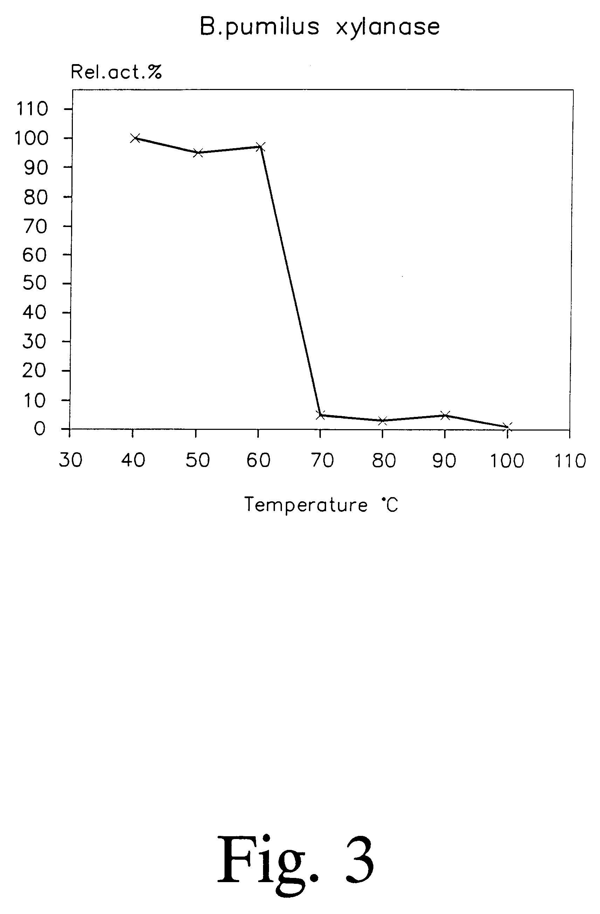

The characterization was performed in the temperature range of from 40 to 100°C. All assays were performed for 20 minutes at pH 6. The profile is presented in Fig. 3.

It appears from this characterization that the curve for temperature profile is very broad, and that the xylanase has no distinct temperature optimum.

The xylanase is found to be nearly as active at 60°C, with around 95% persisting relative activity, as at 40°C. The temperature optimum is found to be lower than 70°C, where only around 5% of the xylanase activity persist. The optimum is within the range of from 35 to 65°C, more specifically from 40 to 60°C.

### EXAMPLE 6

pI characterization

The pI characterization of the xylanase was determined using the purified xylanase preparation according to Examples 1 and 2, and LKB Ampholine PAG plates at pH 3.5 to 9.5.

After electrophoresis, the gel was washed twice for 15 min., once in water, once in a Tris-buffer, pH 9, and then overlayered with a thin coat of detection agar consisting of 0.5% of oat spelt xylan, 1% of agarose, pH 9. The overlayered gel was incubated overnight at 50°C.

The xylanase activity was visualised using Congo Red staining (staining for 10 min. with 0.1% Congo Red, and destained for 2 times 15 min. in 1 M NaCl).

The pI for the purified xylanase was found to be above 8, within a range of from 8.0 to 9.5, around 8.8.

### EXAMPLE 7

Amino acid sequence

Using standard methods for obtaining and sequencing peptides [Findlay & Geisow (Eds.) (1989); Protein sequencing - a practical approach; IRL Press] derived from the B. pumilus xylanase a total of 163 amino acid residues has been identified. In addition, the C-terminal amino acid has been identified as isoleucine.

During direct sequencing of the purified enzyme the N-terminus was found to be partially blocked.

An alignment of the partial sequence of the B. pumilus xylanase to the amino acid sequence of the B. pumilus IPO xylanase [Fukusaki et al. (1984); op. cit.] is shown in Fig. 1. A total of 26 differences is found and shown in bold typing. Positions in the two sequences which are occupied by identical amino acid residues are outlined. In addition to the 26 differences mentioned above there are two amino acid residues less at the C-terminus of the B. pumilus xylanase of the invention than described for the B. pumilus IPO xylanase.

**EXAMPLE 8**

Amino acid composition

The amino acid composition of B. pumilus xylanase was determined after acid hydrolysis [Moore & Stein (1963); Methods Enzymol. 6, 819-831] using an Applied Biosystems 420A amino acid analysis system. The amino acids in the hydrolysis mixture were quantified using reversed phase HPLC after precolumn derivatization with phenylisothiocyanate [Heinrikson & Meredith (1984); Anal. Biochem. 136, 65-74].

The amount of tryptophan was determined spectrophotometrically using the method of Edelhoch [Edelhoch (1967); Biochemistry 6, 1948-1954].

The presence of a single cysteine (Cys) residue in the xylanase was determined through titration with 5,5'-dithiobis-(2-nitrobenzoic acid) [Riddles et al. (1983); Methods Enzymol. 91, 49-60]. The presence of only small amounts of cysteine after acid hydrolysis precludes the presence of disulfide-bridges.

The combined result is shown in Table 1. In addition, a comparison of the amino acid composition of the B. pumilus xylanase with the amino acid composition of the B. pumilus IPO xylanase is shown revealing some differences, especially in the content of serine (Ser) and glycine (Gly).

**EXAMPLE 9**

Treatment of lignocellulosic pulp

An unbleached hardwood kraft pulp is treated with a hemicellulase preparation from Bacillus pumilus - (produced according to Example 1) at the following conditions:

| | |
|---|---|
| Time: | t = 3 hours |
| Temperature: | T = 50°C |
| pH: | pH = 8.0 |
| Consistency: | DS = 10% |
| Dosage: | 715 EXU/kg dry pulp |

After the enzyme treatment the pulp is washed with water and bleached in a three stage bleaching sequence, (D50 C50)E D.

A control is treated in the same way but without addition of enzyme.

Table 2 below shows the kappa numbers of the pulps after the enzyme treatment.

Table 2

| | kappa number | reduction % |
|---|---|---|
| Control | 15.0 | - |
| Bacillus pumilus | 13.6 | 9.2 |

The (D50 C50)E D bleaching stages are performed under the following conditions:

| (DC)-stage | Time: | t = 20 min |
|---|---|---|
| | Temperature: | T = 50°C |
| | Consistency: | DS = 5% |
| | Substitution: | 50% with $ClO_2$ (as active chlorine) |
| | Chlorine multiple: | 0.20 |

| E-stage | Time: | t = 1 hr |
|---|---|---|
| | Temperature: | T = 60°C |
| | Consistency: | DS = 10% |
| | NaOH dosage: | 2.0% (w/w) on dry pulp |

| D-stage | Time: | t = 3 hr |
|---|---|---|
| | Temperature: | T = 70°C |
| | Consistency: | DS = 10% |

Both pulps are bleached to a kappa number of 3.5 after the (D50 C50)E stages. For the control 2.8% (w/w) active chlorine are needed in the (D50 C50)-stage to reach this kappa number. For the enzyme treated pulp only 2.38% (w/w) active chlorine are needed to reach a kappa number of 3.5. This corresponds to a reduction in active chlorine (aCl) of 14.5% for the enzyme treated pulp compared to the control in order to reach the desired kappa number.

In the final D-stage both pulps having a kappa number of 3.5 after (D50 C50)E are bleached to their respective brightness ceilings.

For the enzyme treated pulp a chlorine dioxide dosage of 0.99% (w/w) is needed to bring the pulp to a brightness of 87.2% (ISO) brightness. For the control a dosage of 1.2% (w/w) chlorine dioxide is needed to reach a final brightness of 85% (ISO). This shows that the enzyme treatment makes it possible to reduce the dosage of chlorine dioxide in the final D-stage by 17.5% and at the same time elevates the brightness ceiling of the pulp by a 2.2% ISO brightness.

The enzyme treatment of the pulp neither causes strength nor yield loss.

## EXAMPLE 10

Treatment of lignocellulosic pulp

An $O_2$ delignified kraft pine pulp is treated with the purified hemicellulase preparation from Bacillus pumilus (produced according to Example 1, and purified according to Example 2) at the following conditions:

| Time: | t = 3 hours |
|---|---|
| Temperature: | T = 50°C |
| pH: | pH = 8.5 |
| Consistency: | DS = 10% w/w |
| Dosage: | 815 EXU/kg dry pulp |

The pH was controlled using a Britton-Robinson buffer. After the enzyme treatment the pulp is washed with water and bleached in a two stage bleaching sequence, (D50 C50)E.

A control is treated in the same way but with addition of demineralized water instead of enzyme solution.

Table 3 below shows the kappa numbers of the pulps after the enzyme treatment.

Table 3

| | kappa number | reduction % |
|---|---|---|
| Control | 14.9 | - |
| Bacillus pumilus | 14.1 | 5.4 |

The (D50 C50)E bleaching stages are performed using 50% substitution with chlorine dioxide at the following conditions:

|  | (D50 C50)-Stage | E-Stage |
|---|---|---|
| Temperature | 40 °C | 60 °C |
| Time | 45 min. | 60 min. |
| Consistency | 5 % | 12 % |

The chlorine multiple in the (D50 C50)-Stage was 0.20, similar to 2.98 % active chlorine (aCl). The amount of NaOH applied in the E-stage is adjusted according to the dosage of active chlorine (aCl) in the (D50 C50)-Stage, i.e.

$$0.5(\%Cl + \%ClO_2) + 0.3 = 1.8 \%$$

After the (D50 C50)E stages, the kappa number of the pulp is determined. The results are presented in Table 4 below.

Table 4

|  | kappa number | reduction % |
|---|---|---|
| Control | 3.50 | - |
| Bacillus pumilus | 2.35 | 33 |

The enzyme treatment of the pulp neither causes strength nor yield loss.

This Example demonstrate the bleach boosting effect of a purified enzyme preparation of this invention.

**Claims**

1. A xylanase comprising the partial amino acid sequences according to the lower sequence on Fig. 1 and Seq. ID. Nos. 2-4 of the sequence list, and having immunochemical properties identical or partially identical to those of a xylanase derived from the strain DSM, No. 6124.

2. A xylanase comprising the partial amino acid sequences according to the lower sequence on Fig. 1 and Seq. ID. Nos. 2-4 of the sequence list, and being obtainable from a strain of Bacillus pumilus.

3. The xylanase of claim 2, being obtainable from the strain DSM, No. 6124.

4. The xylanase according to any of claims 1-3, characterized by having a molecular weight of approximately 22 kDa.

5. The xylanase according to any of claims 1-4, further characterized by having pH optimum in the range of from pH 5 to 7; a temperature optimum below 70 °C; and pI in the range of from 8.0 to 9.5.

6. A xylanase comprising the amino acid sequences according to the lower sequence on Fig. 4 and Seq. ID. No. 5-8 of the sequence list.

7. The xylanase of claim 6, obtainable from a xylanase producing strain of Bacillus pumilus.

8. The xylanase of either of claims 6-7, obtainable from B. pumilus, DSM No. 6124.

9. A method of producing a xylanase of any of claims 1-8, comprising cultivation of a strain of B. pumilus, or a mutant or a variant thereof, in a nutrient medium containing assimilable carbon and nitrogen together with other essential nutrient, followed by recovery of the desired enzyme.

10. The method of claim 9, comprising cultivation of the strain B. pumilus, DSM No. 6124, or a mutant or a variant thereof.

11. A method of producing a xylanase according to any of claims 1-8, comprising isolating a DNA fragment encoding the xylanase; combining the DNA fragment with an appropriate expression signal in an appropriate plasmid vector; introducing the plasmid vector into an appropriate host either as an autonomously replicating plasmid or integrated into the chromosome; cultivating the host organism under conditions leading to expression of the xylanase; and recovering the xylanase from the culture medium.

12. The method of claim 11, in which the host organism is an Escherichia coli, or a member of the genus Bacillus, Aspergillus, or Streptomyces.

13. The use of the xylanase according to any of claims 1-8 in the treatment of lignocellulosic pulp.

14. A process according to claim 13 for treatment of lignocellulosic chemical pulp, wherein the lignocellulosic pulp is treated with the xylanase at a pH above 6.5, preferably above 7.5, whereafter the thus treated cellulosic pulp is treated with chlorine at an active chlorine multiple of 0.20 or less in the first chlorination stage.

15. The process of claim 14, wherein the xylanase treatment is performed at temperatures between 40 and 100°C, preferably between 40 and 80°C, more preferred between 50 and 70°C.

16. A process of either of claims 14-15, wherein the xylanase treatment is performed over a period of 15 minutes to 24 hours, preferably between 30 minutes and 5 hours, most preferred between 30 minutes and 3 hours.

17. A process of any of claims 14-16, wherein the xylanase treatment takes place at a consistency of 5-35%, preferably 8-25%, most preferred 8-15%.

18. A process of any of claims 14-17, wherein an (EOP) treatment is introduced between the xylanase treatment and the chlorine treatment.

19. A process of any of claims 14-18, wherein the chlorine multiple is between 0.10 and 0.20.

**Patentansprüche**

1. Xylanase, enthaltend die partiellen Aminosäuresequenzen gemäß der unteren Sequenz von Fig. 1 und die Sequenzen Id. Nr. 2-4 der Sequenzliste, wobei sie immunochemische Eigenschaften aufweist, die identisch oder partiell identisch mit den Eigenschaften einer vom Stamm DSM Nr. 6124 abgeleiteten Xylanase sind.

2. Xylanase, enthaltend die partiellen Aminosäuresequenzen gemäß der unteren Sequenz von Fig. 1 und die Sequenzen Id. Nr. 2-4 der Sequenzliste und erhältlich aus einem Stamm von Bacillus pumilus.

3. Xylanase nach Anspruch 2, erhältlich aus dem Stamm DSM Nr. 6124.

4. Xylanase nach einem der Ansprüche 1-3, gekennzeichnet durch ein Molekulargewicht von etwa 22 kDa.

5. Xylanase nach einem der Ansprüche 1-4, ferner gekennzeichnet durch ein pH-Optimum im Bereich von 5 bis 7, ein Temperaturoptimum unter 70°C und einen pI-Wert im Bereich von 8,0 bis 9,5.

6. Xylanase, umfassend die Aminosäuresequenzen gemäß der unteren Sequenz von Fig. 4 und die Sequenzen Id. Nr. 5-8 der Sequenzliste.

7. Xylanase nach Anspruch 6, erhältlich aus einem Xylanase bildenden Stamm von Bacillus pumilus.

8. Xylanase nach einem der Ansprüche 6 oder 7, erhältlich aus B. pumilus, DSM Nr. 6124.

9. Verfahren zur Herstellung einer Xylanase nach einem der Ansprüche 1-8, umfassend das Züchten eines Stamms von B. pumilus oder einer Mutante oder Variante davon, in einem Nährmedium mit einem Gehalt an assimilierbarem Kohlenstoff und Stickstoff zusammen mit anderen essentiellen Nährstoffen, gefolgt von einer Gewinnung des gewünschten Enzyms.

10. Verfahren nach Anspruch 9, umfassend das Züchten des Stamms B. pumilus, DSM Nr. 6124, oder einer Mutante oder einer Variante davon.

11. Verfahren zur Herstellung einer Xylanase nach einem der Ansprüche 1-8, umfassend das Isolieren eines für die Xylanase kodierenden DNA-Fragments, das Vereinigen des DNA-Fragments mit einem geeigneten Expressionssignal in einem geeigneten Plasmidvektor, das Einführen des Plasmidvektors in einen geeigneten Wirt entweder als autonom replizierendes Plasmid oder integriert in das Chromosom, Züchten des Wirtsorganismus unter Bedingungen, die zur Expression der Xylanase führen und Gewinnen der Xylanase aus dem Kulturmedium.

12. Verfahren nach Anspruch 11, wobei es sich beim Wirtsorganismus um Escherichia coli oder ein Mitglied der Gattung Bacillus, Aspergillus oder Streptomyces handelt.

13. Verwendung der Xylanase nach einem der Ansprüche 1-8 bei der Behandlung von Lignocellulosepulpe.

14. Verfahren nach Anspruch 13 zur Behandlung von chemischer Lignocellulosepulpe, wobei die Lignocellulosepulpe mit der Xylanase bei einem pH-Wert über 6,5 und vorzugsweise über 7,5 behandelt wird, wonach die auf diese Weise behandelte Cellulosepulpe mit Chlor bei einem Vielfachen an aktivem Chlor von 0,20 oder weniger in der ersten Chlorierungsstufe behandelt wird.

15. Verfahren nach Anspruch 14, wobei die Xylanasebehandlung bei Temperaturen zwischen 40 und 100°C, vorzugsweise zwischen 40 und 80°C und insbesondere zwischen 50 und 70°C durchgeführt wird.

16. Verfahren nach einem der Ansprüche 14-15, wobei die Xylanasebehandlung für einen Zeitraum von 15 Minuten bis 24 Stunden, vorzugsweise zwischen 30 Minuten und 5 Stunden und insbesondere zwischen 30 Minuten und 3 Stunden durchgeführt wird.

17. Verfahren nach einem der Ansprüche 14-16, wobei die Xylanasebehandlung bei einer Konsistenz von 5-35%, vorzugsweise 8-25% und insbesondere 8-15% stattfindet.

18. Verfahren nach einem der Ansprüche 14-17, wobei eine (EOP)-Behandlung zwischen der Xylanasebehandlung und der Chlorbehandlung eingeschoben wird.

19. Verfahren nach einem der Ansprüche 14-18, wobei das Chlor-Vielfache zwischen 0,10 und 0,20 liegt.

**Revendications**

1. Xylanase comprenant les séquences partielles d'acides aminés suivant la séquence inférieure de la figure 1 et les séquences ID n° 2 à 4 de la liste de séquences, et ayant des propriétés immunochimiques identiques ou partiellement identiques à celles d'une xylanase dérivée de la souche DSM, n° 6124.

2. Xylanase comprenant les séquences d'acides aminés partielles suivant la séquence inférieure de la figure 1 et les séquences ID n° 2 à 4 de la liste de séquences, et pouvant être obtenue à partir d'une souche de Bacillus pumilus.

3. Xylanase selon la revendication 2, pouvant être obtenue à partir de la souche DSM, n° 6124.

4. Xylanase selon l'une quelconque des revendications 1 à 3, caractérisée en ce qu'elle a un poids moléculaire d'approximativement 22 kDa.

5. Xylanase selon l'une quelconque des revendications 1 à 4, caractérisée, en outre, en ce qu'elle a un optimum de pH dans la gamme de pH 5 à pH 7; un optimum de température inférieur à 70°C; et un pI dans la gamme allant de 8,0 à 9,5.

6. Xylanase comprenant les séquences d'acides aminés suivant la séquence inférieure de la figure 4 et les séquences ID n° 5 à 8 de la liste de séquences.

7. Xylanase selon la revendication 6, pouvant être obtenue à partir d'une souche de Bacillus pumilus produisant une xylanase.

8. Xylanase selon les revendications 6 ou 7, pouvant être obtenue à partir de B. pumilus, DSM n° 6124.

9. Procédé de production d'une xylanase selon l'une quelconque des revendications 1 à 8, comprenant la culture d'une souche de B. pumilus, ou d'un mutant ou d'un variant de celle-ci, dans un milieu nutritif contenant du carbone et de l'azote assimilables associés avec d'autres éléments nutritifs essentiels, suivie par une récupération de l'enzyme désirée.

10. Procédé selon la revendication 9, comprenant une culture de la souche B. pumilus, DSM n° 6124, ou d'un mutant ou d'un variant de celle-ci.

11. Procédé de production d'une xylanase selon l'une quelconque des revendications 1 à 8, consistant à isoler un fragment d'ADN codant pour la xylanase; à combiner le fragment d'ADN avec un signal d'expression approprié dans un vecteur plasmidique approprié; à introduire le vecteur plasmidique dans un hôte approprié soit sous la forme d'un plasmide se répliquant de manière autonome soit intégré dans le chromosome; à cultiver l'organisme hôte dans des conditions aboutissant à l'expression de la xylanase; et à récupérer la xylanase à partir du milieu de culture.

12. Procédé selon la revendication 11, dans lequel l'organisme hôte est un Escherichia coli, ou un membre du genre Bacillus, Aspergillus, ou Streptomyces.

13. Utilisation de la xylanase selon l'une quelconque des revendications 1 à 8 dans le traitement d'une pâte lignocellulosique.

14. Procédé selon la revendication 13, pour le traitement d'une pâte chimique lignocellulosique, dans lequel la pâte lignocellulosique est traitée avec la xylanase à un pH supérieur à 6,5, de préférence supérieur à 7,5, après quoi la pâte lignocellulosique ainsi traitée est traitée avec du chlore sous la forme de multiple de chlore actif de 0,20 ou moins dans le premier stade de chloration.

15. Procédé selon la revendication 14, dans lequel le traitement à la xylanase est réalisé à des températures situées entre 40 et 100°C, de préférence entre 40 et 80°C, de manière plus préférée entre 50 et 70°C.

16. Procédé selon l'une des revendications 14 ou 15, dans lequel le traitement à la xylanase est réalisé sur une période de 15 minutes à 24 heures, de préférence entre 30 minutes et 5 heures, de manière plus préférée entre 30 minutes et 3 heures.

17. Procédé selon l'une quelconque des revendications 14 ou 16, dans lequel le traitement à la xylanase a lieu à une consistance de 5 à 35%, de préférence de 8 à 25%, de manière plus préférée de 8 à 15%.

18. Procédé selon l'une quelconque des revendications 14 à 17, dans lequel un traitement (EOP) est introduit entre le traitement à la xylanase et le traitement au chlore.

19. Procédé selon l'une quelconque des revendications 14 à 18, dans lequel le multiple de chlore est entre 0,10 et 0,20.

EP 0 546 045 B1

```
R T I T N N E M G N H S G Y D Y E L W K D Y G N T S M T L N
E T I Y D N R I G R H T G Y D F E L W K D Y G N T S M I

N G G A F S A G W N N I G N A L F R K G K K F D S T R T H H
        S A S W N N I G N A L F R K G K K F D S T K T H H

Q L G N I S I N Y N A S F N P S G N S Y L C V Y G W T Q S P
Q L G N I D

L A E Y Y I V D S W G T Y R P T G A Y K G S F Y A D G G T Y
        I V E S W G T Y R P T G T Y K W S F Y A D G G T Y

D I Y E T T R V N Q P S I I G I A T F K Q Y W S V R Q T K R
D I Y E T L R V N Q P S I I G D A T F K Q Y V S V R Q T K R

T S G T V S V S A H F R K W E S L G M P M G K M Y E T A F T
T S G T V S V S E H F K K W E G L Y M P M G K M Y E T A L T

V E G Y Q S S G S A N V M T N Q L F I G N
V E G Y R S T G S A N V M T N Q L M I
```

# Fig. 1

pH profile of the B.pumilus xylanase

Fig. 2

B.pumilus xylanase

Fig. 3

**Fig. 4**

```
R T I T N N E M G N H S G Y D Y E L W K D Y G N T S M T L N
E T I   Y D N R I G R H T G Y D F E L W K D Y G N T S M I

N G G A F S A G W N N I G N A L F R K G K K F D S T R T H H
        S A S W N N I G N A L F R K G K K F D S T K T H H

Q L G N I S I N Y N A S F N P S G N S Y L C V Y G W T Q S P
Q L G N I D

L A E Y Y I V D S W G T Y R P T G A Y K G S F Y A D G G T Y
        I V E S W G T Y R P T G T Y K G

D I Y E T T R V N Q P S I I G I A T F K Q Y W S V R Q T K R
            V N Q P S I I G D A T F K Q Y V S V R Q T K R

T S G T V S V S A H F R K W E S L G M P M G K M Y E T A F T T
                H F K K X E G L                         T

V E G Y Q S S G S A N V M T N Q L F I G N
V A G Y
```